# EUROPEAN PATENT APPLICATION

(11) **EP 3 527 257 A2**
(43) Date of publication of application: **21.08.2019**
(21) Application number: 18192290.7
(22) Date of filing: 05.01.2015
(51) Int. Cl.: A61N 1/36

(54) **NEUROMODULATORY SYSTEMS AND METHODS FOR TREATING FUNCTIONAL GASTROINTESTINAL DISORDERS**

(30) Priority: 06.01.2014 US 201461923889 P
(62) Divisional of application: 15701434.1
(71) Applicant: Ohio State Innovation Foundation, Columbus, OH 43201 (US)
(72) Inventor: REZAI, Ali R., Columbus, OH 43215 (US); CHRISTOFI, Fievos L., Lewis Center, OH 43035 (US)
(74) Representative: Gill Jennings & Every LLP

(57) **Abstract**

One aspect of the present disclosure relates to a therapy delivery device for treating a functional gastrointestinal (Gl) disorder in a subject. The device can including a housing, at least one electrode, and a power source. The housing can be configured for placement on the skin of the subject. The at least one electrode can be connected to the housing and configured to deliver an electrical signal to an autonomic nervous system (ANS) target associated with the functional Gl disorder. The ANS nerve target can include one or more mesenteric plexus, a gastric plexus, or a ganglion of the sympathetic nervous system (SNS). The power source can be in electrical communication with the at least one electrode. The functional Gl disorder can be at least one functional dyspepsia, functional constipation, and gastroesophageal reflux disease.

## Description

### RELATED APPLICATION

This application claims the benefit of U.S. Provisional Patent Application Serial No. 61/923,889, filed January 6, 2014, the entirety of which is hereby incorporated by reference for all purposes.

### TECHNICAL FIELD

The present disclosure relates generally to neuromodulatory devices, systems and methods, and more particularly to devices, systems, and methods for treating functional gastrointestinal disorders.

### BACKGROUND

Functional gastrointestinal (GI) and motility disorders are the most common GI disorders in the general population. In fact, about 1 in 4 people in the U.S. have some activity limitation of daily function due to these disorders. The conditions account for about 41 % of GI problems seen by doctors and therapists. The term "functional" is generally applied to disorders where the body's normal activities in terms of the movement of the intestines, the sensitivity of the nerves of the intestines, or the way in which the brain controls some of these functions is impaired. However, there are no structural abnormalities that can be seen by endoscopy, x-ray, or blood tests. Thus, functional GI disorders are identified by the characteristics of the symptoms and infrequently, when needed, limited tests. The Rome diagnostic criteria categorize the functional gastrointestinal disorders and define symptom based diagnostic criteria for each category (*see* Drossman DA, et al. Rome III, the functional gastrointestinal disorders. Gastroenterology. April 2006 Volume 130 Number 5).

### SUMMARY

The present disclosure relates generally to neuromodulatory devices, systems and methods, and more particularly to devices, systems, and methods for treating functional gastrointestinal disorders.

One aspect of the present disclosure relates to a therapy delivery device for treating a functional gastrointestinal (GI) disorder in a subject. The device can comprise a housing, at least one electrode, and a power source. The housing can be configured for placement on the skin of the subject. The at least one electrode can be connected to the housing and configured to deliver an electrical signal to an autonomic nervous system (ANS) target associated with the functional GI disorder. The ANS nerve target can include one or more of a mesenteric plexus, a gastric plexus, or a ganglion of the sympathetic nervous system (SNS). The power source can be in electrical communication with the at least one electrode. The functional GI disorder can be at least one of functional dyspepsia, functional constipation, and gastroesophageal reflux disease.

Another aspect of the present disclosure relates to a closed-loop therapy delivery system for treating a functional GI disorder in a subject. The system can comprise a sensing component, a delivery component, and a controller. The sensing component can be configured to detect at least one physiological parameter associated with the functional GI disorder. The delivery component can be configured for placement on the skin of the subject adjacent an ANS target associated with the functional GI disorder. The ANS nerve target can include one or more of a mesenteric plexus, a gastric plexus, or a ganglion of the SNS. The controller can be configured to automatically coordinate operation of the sensing and delivery components. The controller can be configured to deliver an electrical signal to the delivery component in an amount and for a time sufficient to treat at least one of functional dyspepsia, functional constipation, and gastroesophageal reflux disease.

Another aspect of the present disclosure relates to a closed-loop therapy delivery system for treating a functional GI disorder in a subject. The system can comprise a sensing component, a delivery component, and a controller. The sensing component can be configured to detect at least one physiological parameter associated with the functional GI disorder. The delivery component can be configured for placement in a vessel of the subject at a point substantially adjacent an intraluminal target site of the ANS, the central nervous system (CNS), or both, that is associated with the functional GI disorder. The controller can be configured to automatically coordinate operation of the sensing and delivery components. The controller can be configured to deliver an electrical signal to the delivery component in an amount and for a time sufficient to treat at least one of functional dyspepsia and functional constipation.

### BRIEF DESCRIPTION OF THE DRAWINGS

The foregoing and other features of the present disclosure will become apparent to those skilled in the art to which the present disclosure relates upon reading the following description with reference to the accompanying drawings, in which:
Fig. 1 is schematic illustration showing the cervical and upper thoracic portions of the sympathetic nerve chain and the spinal cord;
Fig. 2 is a schematic illustration of a human spinal cord and associated vertebrae;
Fig. 3 is a schematic illustration showing a closed-loop therapy delivery system for treating a functional gastrointestinal (GI) disorder configured according to one aspect of the present disclosure;

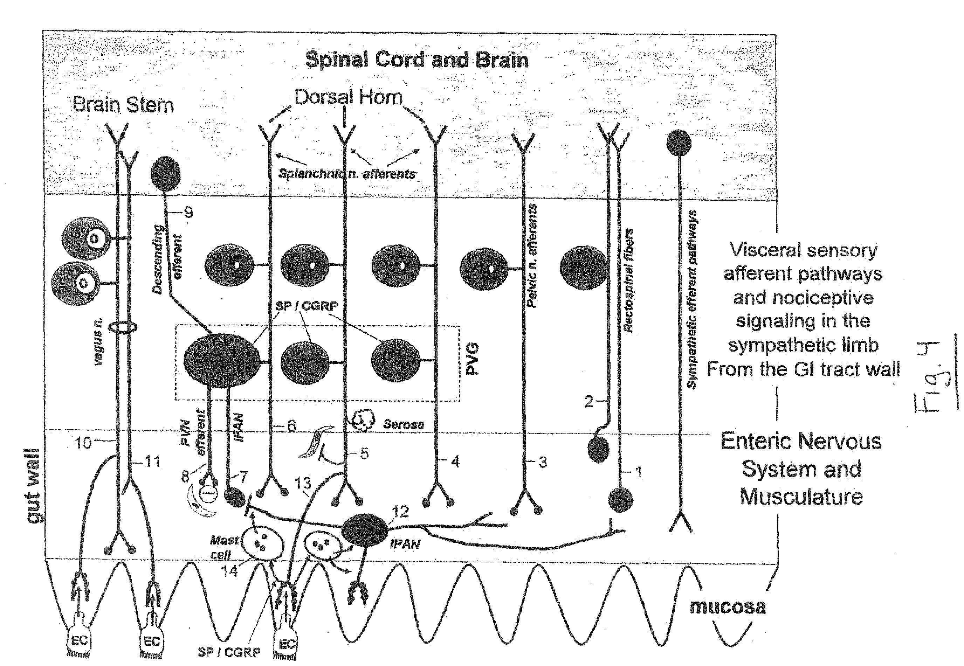

Fig. 4 is a schematic illustration showing the main visceral afferent signaling pathways in the GI tract. Visceral afferent signaling pathways (1-12) transmit pain or physiologic information from the gastrointestinal tract to the spinal cord and brain. Depicted are the sympathetic spinal afferents carrying information about pain via the dorsal root ganglia (DRG) to the dorsal horns (DH) of the spinal cord. From there, second order neurons transmit pain to higher centers in the brain. Rectospinal afferents transmit information from the gut wall to the spinal cord as the name implies. Vagal afferents transmit physiologic information to the brain stem and higher centers from the gut wall via the nodose ganglia (NG) and jugular ganglia (JG). The prevertebral ganglia (PVG) orchestrate reflex arcs from one region of the intestinal tract to another, and are involved in entero-enteric motor (peristaltic and secretory) reflexes as well as reflexes that reduce the overall tone of smooth muscles of the gut. Sympathetic spinal afferents carried in the splanchnic nerves send collaterals to the prevertebral ganglia (PVG), *i.e*., the inferior mesenteric ganglion (IMG), superior mesenteric ganglion (SMG) and celiac ganglion (CG). Release of SP or CGRP from these collaterals can modulate the neural activity in PVG and, hence, influence entero-enteric reflexes. Intrinsic to the gut is the enteric nervous system (ENS) and musculature that regulates all digestive and motor functions including peristalsis, motility, transit, secretions, transport, vasomotor and neuro-immune functions. Interactions between the ENS, PVG, DRG, spinal cord and brain, and alterations in activity at any level of these neural circuit pathways can lead to visceral pain sensation from the stomach and intestines, or abnormal motility, transit, or secretions associated with gastroparesis, bloating, GI discomfort, diarrhea or constipation, as occurs in irritable bowel syndrome or functional dyspepsia. Immune-neural interactions with intestinalfugal afferent neurons (IFANs) projecting to PVG, intrinsic afferent neurons in the ENS or any of the visceral afferents from the gut can exacerbate visceral pain signaling and abbarrent GI motor behaviors. Afferent collaterals in the gut, PVG or spinal cord can exacerbate painful sensations carried through sensitized afferents in FGID's, Mast cells (MC) are important immune cells involved in immune-neural modulation, and CGRP/SP release from collaterals can activate these cells in FGID's;
Fig. 5 is a process flow diagram illustrating a method for treating a functional GI disorder according to another aspect of the present disclosure;
Fig. 6 is a schematic illustration showing a transcutaneous neuromodulatory device constructed in accordance with another aspect of the present disclosure; and
Figs. 7A-B are schematic illustrations showing alternative transcutaneous neuromodulatory devices constructed in accordance with other aspects of the present disclosure.

### DETAILED DESCRIPTION

### Definitions

Unless defined otherwise, all technical and scientific terms used herein have the same meaning as is commonly understood by one of skill in the art to which the present disclosure pertains.

In the context of the present disclosure, the term "autonomic nervous tissue" can refer to any tissues of the sympathetic nervous system (SNS) or the parasympathetic nervous system (PNS) including, but not limited to, neurons, axons, fibers, tracts, nerves, plexus, afferent plexus fibers, efferent plexus fibers, ganglia, pre-ganglionic fibers, post-ganglionic fibers, afferents, efferents, and combinations thereof. In some instances, autonomic nervous tissue can comprise an autonomic nervous system (ANS) nerve target.

As used herein, the terms "epidural space" or "spinal epidural space" can refer to an area in the interval between the dural sheath and the wall of the spinal canal. In some instances, at least a portion of a therapy delivery device or a therapy delivery system may be implanted in the epidural space.

As used herein, the term "subdural" can refer to the space between the dura mater and arachnoid membrane. In some instances, at least a portion of a therapy delivery device or a therapy delivery system may be implanted in the subdural space.

As used herein, the phrase "spinal nervous tissue" can refer to nerves, neurons, neuroglial cells, glial cells, neuronal accessory cells, nerve roots, nerve fibers, nerve rootlets, parts of nerves, nerve bundles, mixed nerves, sensory fibers, motor fibers, dorsal root, ventral root, dorsal root ganglion, spinal ganglion, ventral motor root, general somatic afferent fibers, general visceral afferent fibers, general somatic efferent fibers, general visceral efferent fibers, grey matter, white matter, the dorsal column, the lateral column, and/or the ventral column associated with the spinal cord. In some instances, spinal nervous tissue can comprise a central nervous system (CNS) nerve target.

As used herein, the term "subject" can be used interchangeably with the term "patient" and refer to any warm-blooded organism including, but not limited to, human beings, pigs, rats, mice, dogs, goats, sheep, horses, monkeys, apes, farm animals, livestock, rabbits, cattle, etc.

As used herein, the terms "modulate" or "modulating" with reference to an autonomic nervous tissue or spinal nervous tissue can refer to causing a change in neuronal activity, chemistry and/or metabolism. The change can refer to an increase, decrease, or even a change in a pattern of neuronal activity. The terms may refer to either excitatory or inhibitory stimulation, or a combination thereof, and may be at least electrical, magnetic, ultrasound, optical, chemical, or a combination of two or more of these. The terms "modulate" or "modulating" can also be used to refer to a masking, altering, overriding, or restoring of neuronal activity.

As used herein, the terms "substantially blocked" or "substantially block" when used with reference to nervous tissue activity can refer to a complete (*e.g*., 100%) or partial inhibition (*e.g*., less than 100%, such as about 90%, about 80%, about 70%, about 60%, or less than about 50%) of nerve conduction through the nervous tissue.

As used herein, the term "activity" when used with reference to autonomic or spinal nervous tissue can, in some instances, refer to the ability of a nerve, neuron, or fiber to conduct, propagate, and/or generate an action potential. In other instances, the term can refer to the frequency at which a nerve or neuron is conducting, propagating, and/or generating one or more action potentials at a given moment in time. In further instances, the term can refer to the frequency at which a nerve or neuron is conducting, propagating, and/or generating one or more action potentials over a given period of time (*e.g*., seconds, minutes, hours, days, etc.).

As used herein, the term "electrical communication" can refer to the ability of an electric field generated by an electrode or electrode array to be transferred, or to have a neuromodulatory effect, within and/or on autonomic or spinal nervous tissue.

As used herein, the term "functional gastrointestinal disorder" can refer to a disease or condition having one or more gastrointestinal (GI) symptoms or combinations of GI symptoms of a chronic or recurrent nature that do not have an identified underlying pathophysiology (*e.g*., are not attributable to anatomic or biochemical defects). In the absence of any objective marker(s), the identification and classification of functional GI disorders can be based on symptoms. Examples of such symptoms can include abdominal pain, early satiety, nausea, bloating, distention, and various symptoms of disordered defecation. In some instances, such classification can be based on the Rome diagnostic criteria. Non-limiting examples of functional GI disorders can include visceral pain, irritable bowel syndrome (IBS), functional dyspepsia, functional constipation, functional diarrhea, gastroesophageal reflux disease (GERD), and functional abdominal bloating, as well as those listed below.

As used herein, the terms "treat" or "treating" can refer to therapeutically regulating, preventing, improving, alleviating the symptoms of, and/or reducing the effects of a functional GI disorder. As such, treatment also includes situations where a functional GI disorder, or at least symptoms associated therewith, is completely inhibited, *e.g*., prevented from happening or stopped (*e.g*., terminated) such that the subject no longer suffers from the functional GI disorder, or at least the symptoms that characterize the functional GI disorder. In some instances, the terms can refer to improving or normalizing at least one function of an organ or organ tissue affected by an imbalanced sympathetic and/or parasympathetic input.

As used herein, the term "in communication" can refer to at least a portion of a therapy delivery device or therapy delivery system being adjacent, in the general vicinity, in close proximity, or directly next to and/or directly on an ANS nerve target (*e.g*., autonomic nervous tissue) or CNS nerve target (*e.g*., spinal nervous tissue) associated with a functional GI disorder. In some instances, the term can mean that at least a portion of a therapy delivery device or therapy delivery system is "in communication" with an ANS and/or CNS nerve target if application of a therapy signal (*e.g*., an electrical and/or chemical signal) thereto results in a modulation of neuronal activity to elicit a desired response, such as modulation of a sign or symptom associated with a functional GI disorder.

As used herein, the singular forms "a," "an" and "the" can include the plural forms as well, unless the context clearly indicates otherwise. It will be further understood that the terms "comprises" and/or "comprising," as used herein, can specify the presence of stated features, steps, operations, elements, and/or components, but do not preclude the presence or addition of one or more other features, steps, operations, elements, components, and/or groups thereof.

As used herein, the term "and/or" can include any and all combinations of one or more of the associated listed items.

As used herein, phrases such as "between X and Y" and "between about X and Y" can be interpreted to include X and Y.

As used herein, phrases such as "between about X and Y" can mean "between about X and about Y."

As used herein, phrases such as "from about X to Y" can mean "from about X to about Y."

It will be understood that when an element is referred to as being "on," "attached" to, "connected" to, "coupled" with, "contacting," etc., another element, it can be directly on, attached to, connected to, coupled with or contacting the other element or intervening elements may also be present. In contrast, when an element is referred to as being, for example, "directly on," "directly attached" to, "directly connected" to, "directly coupled" with or "directly contacting" another element, there are no intervening elements present. It will also be appreciated by those of skill in the art that references to a structure or feature that is disposed "directly adjacent" another feature may have portions that overlap or underlie the adjacent feature, whereas a structure or feature that is disposed "adjacent" another feature may not have portions that overlap or underlie the adjacent feature.

Spatially relative terms, such as "under," "below," "lower," "over," "upper" and the like, may be used herein for ease of description to describe one element or feature's relationship to another element(s) or feature(s) as illustrated in the figures. It will be understood that the spatially relative terms can encompass different orientations of a device in use or operation, in addition to the orientation depicted in the figures. For example, if a device in the figures is inverted, elements described as "under" or "beneath" other elements or features would then be oriented "over" the other elements or features.

It will be understood that, although the terms "first," "second," etc. may be used herein to describe various elements, these elements should not be limited by these terms. These terms are only used to distinguish one element from another. Thus, a "first" element discussed below could also be termed a "second" element without departing from the teachings of the present disclosure. The sequence of operations (or steps) is not limited to the order presented in the claims or figures unless specifically indicated otherwise.

### Overview

A brief discussion of the pertinent neurophysiology is provided to assist the reader with understanding certain aspects of the present disclosure.

### The autonomic nervous system (ANS)

The nervous system is divided into the somatic nervous system and the ANS. In general, the somatic nervous system controls organs under voluntary control (*e.g*., skeletal muscles) and the ANS controls individual organ function and homeostasis. For the most part, the ANS is not subject to voluntary control. The ANS is also commonly referred to as the visceral or automatic system.

The ANS can be viewed as a "real-time" regulator of physiological functions which extracts features from the environment and, based on that information, allocates an organism's internal resources to perform physiological functions for the benefit of the organism, *e.g*., responds to environment conditions in a manner that is advantageous to the organism. The ANS acts through a balance of its two components: the sympathetic nervous system (SNS) and the parasympathetic nervous system (PNS), which are two anatomically and functionally distinct systems. Both of these systems include myelinated preganglionic fibers which make synaptic connections with unmyelinated postganglionic fibers, and it is these fibers which then innervate the effector structure. These synapses usually occur in clusters called ganglia. Most organs are innervated by fibers from both divisions of the ANS, and the influence is usually opposing (*e.g*., the vagus nerve slows the heart, while the sympathetic nerves increase its rate and contractility), although it may be parallel (*e.g*., as in the case of the salivary glands). Each of these is briefly reviewed below.

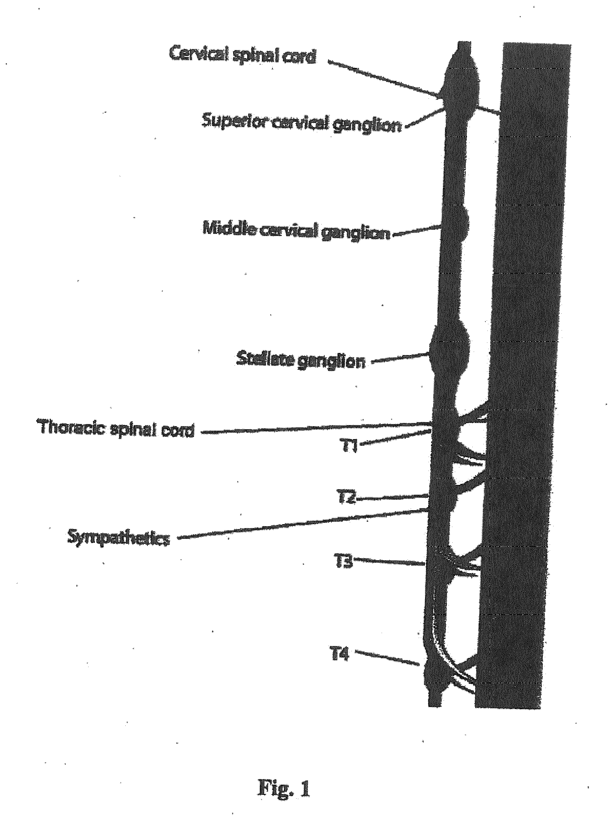

The SNS is the part of the ANS comprising nerve fibers that leave the spinal cord in the thoracic and lumbar regions and supply viscera and blood vessels by way of a chain of sympathetic ganglia (also referred to as the sympathetic chain, sympathetic trunk or the gangliated cord) running on each side of the spinal column, which communicate with the central nervous system via a branch to a corresponding spinal nerve. The sympathetic trunks extend from the base of the skull to the coccyx. The cephalic end of each is continued upward through the carotid canal into the skull, and forms a plexus on the internal carotid artery; the caudal ends of the trunks converge and end in a single ganglion, the ganglion impar, placed in front of the coccyx. As partly shown in Fig. 1, the ganglia of each trunk are distinguished as cervical, thoracic, lumbar, and sacral and, except in the neck, they closely correspond in number to the vertebrae.

The SNS controls a variety of autonomic functions including, but not limited to, control of movement and secretions from viscera and monitoring their physiological state, stimulation of the sympathetic system inducing, *e.g*., the contraction of gut sphincters, heart muscle and the muscle of artery walls, and the relaxation of gut smooth muscle and the circular muscles of the iris. The chief neurotransmitter in the SNS is adrenaline, which is liberated in the heart, visceral muscle, glands and internal vessels, with acetylcholine acting as a neurotransmitter at ganglionic synapses and at sympathetic terminals in skin and skeletal muscles. The actions of the SNS tend to be antagonistic to those of the PNS.

The neurotransmitter released by the post-ganglionic neurons is nonadrenaline (also called norepinephrine). The action of noradrenaline on a particular structure, such as a gland or muscle, is excitatory in some cases and inhibitory in others. At excitatory terminals, ATP may be released along with noradrenaline. Activation of the SNS may be characterized as general because a single pre-ganglionic neuron usually synapses with many post-ganglionic neurons, and the release of adrenaline from the adrenal medulla into the blood ensures that all the cells of the body will be exposed to sympathetic stimulation even if no post-ganglionic neurons reach them directly.

The PNS is the part of the ANS controlling a variety of autonomic functions including, but not limited to, involuntary muscular movement of blood vessels and gut and glandular secretions from eye, salivary glands, bladder, rectum and genital organs. The vagus nerve is part of the PNS. Parasympathetic nerve fibers are contained within the last five cranial nerves and the last three spinal nerves and terminate at parasympathetic ganglia near or in the organ they supply. The actions of the PNS are broadly antagonistic to those of the SNS - lowering blood pressure, slowing heartbeat, stimulating the process of digestion etc. The chief neurotransmitter in the PNS is acetylcholine. Neurons of the parasympathetic nervous system emerge from the brainstem as part of the Cranial nerves III, VII, IX and X (vagus nerve) and also from the sacral region of the spinal cord via Sacral nerves. Because of these origins, the PNS is often referred to as the "craniosacral outflow".

In the PNS, both pre- and post-ganglionic neurons are cholinergic (*i.e*., they utilize the neurotransmitter acetylcholine). Unlike adrenaline and noradrenaline, which the body takes around 90 minutes to metabolize, acetylcholine is rapidly broken down after release by the enzyme cholinesterase. As a result the effects are relatively brief in comparison to the SNS.

Each pre-ganglionic parasympathetic neuron synapses with just a few post-ganglionic neurons, which are located near, or in, the effector organ, a muscle or gland. As noted above, the primary neurotransmitter in the PNS is acetylcholine such that acetylcholine is the neurotransmitter at all the pre- and many of the post-ganglionic neurons of the PNS. Some of the post-ganglionic neurons, however, release nitric oxide as their neurotransmitter.

### The central nervous system (CNS)

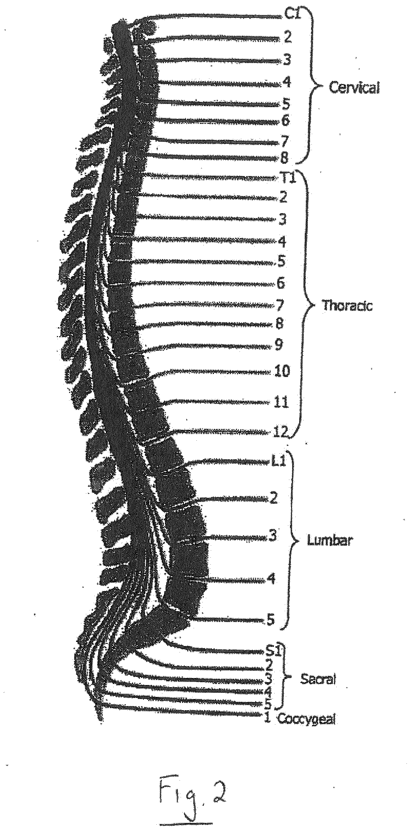

The spinal cord (Fig. 2) is part of the CNS, which extends caudally and is protected by the bony structures of the vertebral column. It is covered by the three membranes of the CNS, *i.e*., the dura mater, arachnoid and the innermost pia mater. In most adult mammals, it occupies only the upper two-thirds of the vertebral canal as the growth of the bones composing the vertebral column is proportionally more rapid than that of the spinal cord. According to its rostrocaudal location, the spinal cord can be divided into four parts: cervical; thoracic; lumbar; and sacral. Two of these are marked by an upper (cervical) and a lower (lumbar) enlargement.

Alongside the median sagittal plane, the anterior and the posterior median fissures divide the cord into two symmetrical portions, which are connected by the transverse anterior and posterior commissures. On either side of the cord the anterior lateral and posterior lateral fissures represent the points where the ventral and dorsal rootlets (later roots) emerge from the cord to form the spinal nerves. Unlike the brain, in the spinal cord the grey matter is surrounded by the white matter at its circumference. The white matter is conventionally divided into the dorsal, dorsolateral, lateral, ventral and ventrolateral funiculi.

Each half of the spinal grey matter is crescent-shaped, although the arrangement of the grey matter and its proportion to the white matter varies at different rostrocaudal levels. The grey matter can be divided into the dorsal horn, intermediate grey, ventral horn, and a centromedial region surrounding the central canal (central grey matter). The white matter gradually ceases towards the end of the spinal cord and the grey matter blends into a single mass (conus terminalis) where parallel spinal roots form the so-called cauda equine.

The present disclosure relates generally to neuromodulatory devices, systems and methods, and more particularly to devices, systems, and methods for treating functional GI disorders. The ANS regulates the intrinsic function and balance of each body organ and maintains homeostasis and balance of the GI system. Neuromodulation of the ANS is a precise, controlled, and highly targeted approach to influence and impact the function and dysfunction in humans. Neuromodulation according to the present disclosure can improve the function, activate, inhibit, modulate, and impact the intrinsic autonomic tone, as well as normalize or regulate the function and sympathetic/parasympathetic output to the GI system, which is impacted in functional GI disorders. As described in detail below, the present disclosure can advantageously provide, in some instances, devices, systems, and methods for uncoupling dysfunctional nerve signals from the brain to the ANS (as well as ascending signals into the CNS), as well as dysfunctional nerve signals from the ANS to peripheral tissues (*e*.*g*., tissues and organs associated with the GI system) to effectively normalize or regulate the ANS (*e.g*., the SNS). In some instances, these effects are anticipated on the basis of the close interactions between intrinsic and extrinsic afferent reflexes coordinating GI sensory/motor functions, as well as visceral afferent signaling to the brain and back. In addition, efferent pathways of the ANS can play important roles in brian-gut interactions and contribute to GI symptoms. By employing such devices, systems and methods, the present disclosure can treat functional GI disorders.

### Therapy Delivery Devices and Systems

In one aspect, the present disclosure includes various therapy delivery devices (not shown) and related systems configured to treat one or more functional GI disorders in a subject. In some instances, therapy delivery devices that may be used to practice the present disclosure may be positioned substantially adjacent (*e.g*., directly adjacent) an intraluminal target site of the ANS, the CNS, or both, that is associated with a functional GI disorder. In other instances, therapy delivery devices used to practice the present disclosure can comprise an external device, *e.g*., positioned on the skin of a subject substantially adjacent (*e.g*., directly adjacent) an intraluminal target site of the ANS, the CNS, or both, that is associated with a functional GI disorder. Therapy delivery devices can be temporarily or permanently implanted within, on, or otherwise associated with a subject suffering from, afflicted by, or suspected of having a functional GI disorder.

Therapy delivery devices of the present disclosure can be configured to deliver various types of therapy signals to ANS and/or CNS nerve targets. For example, therapy delivery devices of the present disclosure can be configured to deliver only electrical energy, only magnetic energy, only a pharmacological or biological agent, or a combination thereof. In one example, therapy delivery devices of the present disclosure can comprise at least one electrode and an integral or remote power source, which is in electrical communication with the one or more electrodes and configured to produce one or more electrical signals (or pulses). In another example, therapy delivery devices can include a pharmacological or biological agent reservoir, a pump, and a fluid dispensing mechanism. Non-limiting examples of pharmacological and biological agents can include chemical compounds, drugs (*e.g*., prazosin, clonidine), nucleic acids, polypeptides, stem cells, toxins (*e*.*g*., botulinum), as well as various energy forms, such as ultrasound, radiofrequency (continuous or pulsed), magnetic waves, cryotherapy, and the like. In yet another example, therapy delivery devices can be configured to deliver magnetic nerve stimulation with desired field focality and depth of penetration. One skilled in the art will appreciate that combinations of the therapy delivery devices above configurations are also included within the scope of the present disclosure.

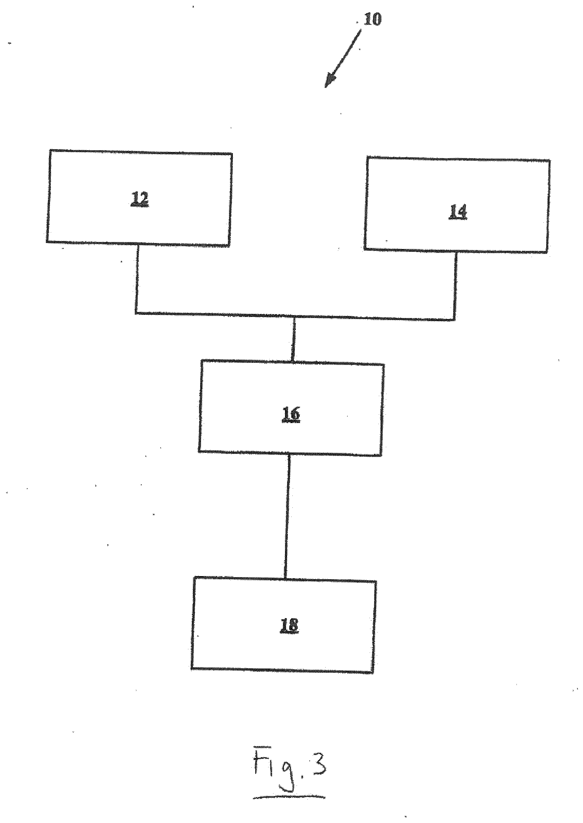

In some instances, therapy delivery devices can comprise a stimulator (or inhibitor), such as an electrode, a controller or programmer, and one or more connectors (*e.g*., leads) for connecting the stimulating (or inhibiting) device to the controller. In one example, which is described in further detail below, the present disclosure can include a closed-loop therapy delivery system 10 (Fig. 3) for treating a functional GI disorder. As shown in Fig. 3, the therapy delivery system 10 can include a sensing component 12, a delivery component 14, a controller 16, and a power source 18. Each of the sensing component 12, delivery component 14, controller 16, and power source 18 can be in electrical communication with one another (*e.g*., via a physical connection, such as a lead, or a wireless link). In some instances, each of the sensing and delivery components 12 and 14 can comprise an electrode. In other instances, the delivery component 14 can comprise a coil configured to deliver magnetic stimulation. In further describing representative electrodes, which are described in the singular, it will be apparent that more than one electrode may be used as part of a therapy delivery device. Accordingly, the description of a representative electrode suitable for use in the therapy delivery devices of the present disclosure is applicable to other electrodes that may be employed.

An electrode can be controllable to provide output signals that may be varied in voltage, frequency, pulse-width, current and intensity. The electrode can also provide both positive and negative current flow from the electrode and/or is capable of stopping current flow from the electrode and/or changing the direction of current flow from the electrode. In some instances, therapy delivery devices can include an electrode that is controllable, *i.e*., in regards to producing positive and negative current flow from the electrode, stopping current flow from the electrode, changing direction of current flow from the electrode, and the like. In other instances, the electrode has the capacity for variable output, linear output and short pulse-width, as well as paired pulses and various waveforms (*e*.*g*., sine wave, square wave, and the like).

The power source 18 can comprise a battery or generator, such as a pulse generator that is operatively connected to an electrode via the controller 16. The power source 18 can be configured to generate an electrical signal or signals. In one example, the power source 18 can include a battery that is rechargeable by inductive coupling. The power source 18 may be positioned in any suitable location, such as adjacent the electrode (*e.g*., implanted adjacent the electrode), or a remote site in or on the subject's body or away from the subject's body in a remote location. An electrode may be connected to the remotely positioned power source 18 using wires, *e.g*., which may be implanted at a site remote from the electrode(s) or positioned outside the subject's body. In one example, an implantable power source 18 analogous to a cardiac pacemaker may be used.

The controller 16 can be configured to control the pulse waveform, the signal pulse width, the signal pulse frequency, the signal pulse phase, the signal pulse polarity, the signal pulse amplitude, the signal pulse intensity, the signal pulse duration, and combinations thereof of an electrical signal. In other instances, the controller 16 can be configured to control delivery of magnetic energy or stimulation to the delivery component 14. The controller 16 may be used to convey a variety of currents and voltages to one or more electrodes and thereby modulate the activity of a target sympathetic nervous tissue. The controller 16 may be used to control numerous electrodes independently or in various combinations as needed to provide stimulation or inhibition of nerve activity. In some instances, an electrode may be employed that includes its own power source, *e.g*., which is capable of obtaining sufficient power for operation from surrounding tissues in the subject's body, or which may be powered by bringing a power source 18 external to the subject's body into contact with the subject's skin, or which may include an integral power source.

The electrical signal (or signals) delivered by the controller 16 to the delivery component 14 may be constant, varying and/or modulated with respect to the current, voltage, pulse-width, cycle, frequency, amplitude, and so forth. For example, a current may range from about 0.001 to about 1000 microampere (mA) and, more specifically, from about 0.1 to about 100 mA. Similarly, the voltage may range from about 0.1 millivolt to about 25 volts, or about 0.5 to about 4000 Hz, with a pulse-width of about 10 to about 1000 microseconds. In one example, the electrical signal can be oscillatory. The type of stimulation may vary and involve different waveforms known to the skilled artisan. For example, the stimulation may be based on the H waveform found in nerve signals (*i.e*., Hoffinan Reflex). In another example, different forms of interferential stimulation may be used.

To increase nerve activity in a portion of the ANS, for example, voltage or intensity may range from about 1 millivolt to about 1 volt or more, *e.g*., 0.1 to about 50 mA or volts (*e*.*g*., from about 0.2 volts to about 20 volts), and the frequency may range from about 1 Hz to about 10,000 Hz, *e.g*., about 1 Hz to about 1000 Hz (*e*.*g*., from about 2 Hz to about 100 Hz). In some instances, pure DC and/or AC voltages may be employed. The pulse-width may range from about 1 microsecond to about 10,000 microseconds or more, *e.g*., from about 10 microseconds to about 2000 microseconds (*e.g*., from about 15 microseconds to about 1000 microseconds). The electrical signal may be applied for at least about 1 millisecond or more, *e.g*., about 1 second (*e.g*., about several seconds). In some instances, stimulation may be applied for as long as about 1 minute or more, *e.g*., about several minutes or more (*e.g*., about 30 minutes or more).

To decrease activity in a portion of the ANS, for example, voltage or intensity may range from about 1 millivolt to about 1 volt or more, *e.g*., 0.1 to about 50 mA or volts (*e.g*., from about 0.2s volt to about 20 volts), and the frequency may range from about 1 Hz to about 2500 Hz, *e.g*., about 50 Hz to about 2500 Hz. In one example, an electrical signal can have a frequency range of about 10,000 Hz or greater (*e.g*., high frequency stimulation) to effectively block nerve conduction. In some instances, pure DC and/or AC voltages may be employed. The pulse-width may range from about 1 microseconds to about 10,000 microseconds or more, *e.g*., from about 10 microseconds to about 2000 microseconds (*e.g*., from about 15 microseconds to about 1000 microseconds). The electrical signal may be applied for at least about 1 millisecond or more, *e.g*., about 1 second (*e.g*., about several seconds). In some instances, the electrical energy may be applied for as long as about 1 minute or more, *e.g*., about several minutes or more (*e.g*., about 30 minutes or more may be used).

The electrode may be mono-polar, bipolar or multi-polar. To minimize the risk of an immune response triggered by the subject against the therapy delivery device, and also to minimize damage thereto (*e.g*., corrosion from other biological fluids, etc.), the electrode (and any wires and optional housing materials) can be made of inert materials, such as silicon, metal, plastic and the like. In one example, a therapy delivery device can include a multi-polar electrode having about four exposed contacts (*e.g*., cylindrical contacts).

As discussed above, the controller 16 (or a programmer) may be associated with a therapy delivery device. The controller 16 can include, for example, one or more microprocessors under the control of a suitable software program. Other components of a controller 16, such as an analog-to-digital converter, etc., will be apparent to those of skill in the art. In some instances, the controller 16 can be configured to record and store data indicative of the intrinsic autonomic tone or activity in the subject. Therefore, the controller 16 can be configured to apply one or more electrical signals to the delivery component 14 when the intrinsic autonomic tone or activity of a subject increases or decreases above a certain threshold value (or range of values), such as a normal or baseline level.

Therapy delivery devices can be pre-programmed with desired stimulation parameters. Stimulation parameters can be controllable so that an electrical signal may be remotely modulated to desired settings without removal of the electrode from its target position. Remote control may be performed, *e.g*., using conventional telemetry with an implanted power source 18, an implanted radiofrequency receiver coupled to an external transmitter, and the like. In some instances, some or all parameters of the electrode may be controllable by the subject, *e.g*., without supervision by a physician. In other instances, some or all parameters of the electrode may be automatically controllable by a controller 16.

In one example, the therapy delivery device can be configured for intravascular or intraluminal placement or implantation. In some instances, a therapy delivery device configured for intravascular or intraluminal placement or implantation can be configured in an identical or similar manner as the expandable electrode disclosed in U.S. Patent Application Serial No. 11/641,331 to Greenberg et al. (hereinafter, "the '331 application"). In one example, the therapy delivery device can be configured for intravascular or intraluminal placement or implantation at an implantation site that is adjacent, or directly adjacent, an intraluminal target site of the ANS, the CNS, or both.

In yet another example, the therapy delivery device can be configured for transcutaneous neuromodulation. In some instances, transcutaneous neuromodulation can include positioning a delivery component (*e.g*., an electrode or magnetic coil) on a skin surface so that a therapy signal (*e.g*., an electrical signal or magnetic field) can be delivered to an ANS nerve target, a CNS nerve target, or both. Transcutaneous neuromodulation can additionally include partially transcutaneous methods (*e.g*., using a fine, needle-like electrode to pierce the epidermis). In other instances, a surface electrode (or electrodes) or magnetic coil can be placed into electrical contact with an ANS nerve target and/or a CNS nerve target associated with a functional GI disorder. Non-limiting examples of transcutaneous neuromodulation devices that may be used for treating functional GI disorders are discussed below.

In one example, an electrical signal used for transcutaneous neuromodulation may be constant, varying and/or modulated with respect to the current, voltage, pulse-width, cycle, frequency, amplitude, and so forth (*e.g*., the current may be between about 1 to 100 microampere), about 10 V (average), about 1 to about 1000 Hz or more, with a pulse-width of about 250 to about 500 microseconds.

In another example, the present disclosure can include a therapy delivery device or system configured for transcutaneous neuromodulation using magnetic stimulation. A magnetic stimulation device or system can generally include a pulse generator (*e.g*., a high current pulse generator) and a stimulating coil capable of producing magnetic pulses with desired field strengths. Other components of a magnetic stimulation device can include transformers, capacitors, microprocessors, safety interlocks, electronic switches, and the like. In operation, the discharge current flowing through the stimulating coil can generate the desired magnetic field or lines of force. As the lines of force cut through tissue (*e.g*., neural tissue), a current is generated in that tissue. If the induced current is of sufficient amplitude and duration such that the cell membrane is depolarized, nervous tissue will be stimulated in the same manner as conventional electrical stimulation. It is therefore worth noting that a magnetic field is simply the means by which an electrical current is generated within the nervous tissue, and that it is the electrical current, and not the magnetic field, which causes the depolarization of the cell membrane and thus stimulation of the target nervous tissue. Thus, in some instances, advantages of magnetic over electrical stimulation can include: reduced or sometimes no pain; access to nervous tissue covered by poorly conductive structures; and stimulation of nervous tissues lying deeper in the body without requiring invasive techniques or very high energy pulses.

Therapy delivery devices can be part of an open- or closed-loop system. In an open-loop system, for example, a physician or subject may, at any time, manually or by the use of pumps, motorized elements, etc., adjust treatment parameters, such as pulse amplitude, pulse-width, pulse frequency, duty cycle, dosage amount, type of pharmacological or biological agent, etc. Alternatively, in a closed-loop system 10 (as discussed above), treatment parameters (*e.g*., electrical signals) may be automatically adjusted in response to a sensed physiological parameter or a related symptom or sign indicative of the extent and/or presence of a functional GI disorder. In a closed-loop feedback system 10, a sensing component 12 can comprise a sensor (not shown in detail) that senses a physiological parameter associated with a functional GI disorder can be utilized. More detailed descriptions of sensors that may be employed in closed-loop systems, as well as other examples of sensors and feedback control techniques that may be employed as part of the present disclosure are disclosed in U.S. Patent No. 5,716,377. One or more sensing components 12 can be implanted on or in any tissue or organ of a subject. For example, a sensing component 12 can be implanted in or on a component of the ANS, such as nerves, ganglia, afferents or efferents, or the spinal cord. Alternatively or additionally, a sensing component 12 can be implanted on or in a body organ and/or an anatomical connection thereof

It should be appreciated that implementing a therapy delivery device as part of a closed-loop system can include placing or implanting a therapy delivery device on or within a subject at an ANS and/or CNS nerve target, sensing a physiological parameter associated with a functional GI disorder, and then activating the therapy delivery device to apply an electrical signal to adjust application of the electrical signal to the ANS and/or CNS nerve target in response to the sensor signal. In some instances, such physiological parameters can include any characteristic, sign, symptom, or function associated with the functional GI disorder, such as a chemical moiety or nerve activity (*e.g*., electrical activity). Examples of such chemical moieties and nerve activities can include the activity of autonomic ganglia (or an autonomic ganglion), the activity of a spinal cord segment or spinal nervous tissue associated therewith, protein concentrations (*e*.*g*., BDNF, IL-1β, KC/GRO, NGAL, TIMP-1, TWEAK, etc.), electrochemical gradients, hormones, neuroendocrine markers (*e.g*., corticosterone and norepinephrine), electrolytes, laboratory values, vital signs (*e.g*., blood pressure), markers of locomotor activity, inflammatory markers, or other signs and biomarkers associated with functional GI disorders.

### Methods

Another aspect of the present disclosure includes methods for treating a functional GI disorder in a subject. Functional GI disorders (FGIDs) represent a highly prevalent group of heterogeneous disorders, and their diagnosis is based on symptoms in the absence of a reliable structural or biochemical abnormality as noted previously. IBS, for example, is a disorder that leads to debilitating symptoms that include abdominal pain, cramping, discomfort, bloating and changes in bowel movements (diarrhea, constipation or alternating diarrhea/constipation). In patients with IBS, heightened pain sensitivity is observed in response to experimental visceral stimulation, and such patients are said to have visceral pain hypersensitivity.

Fig. 4 illustrates the main visceral afferent signaling pathways in the gastrointestinal tract. It includes intrinsic primary afferent neurons of the intrinsic nervous system of the gut, referred to as the enteric nervous system (ENS), intestinofugal afferent neurons (IFANs) transmitting information from the ENS to prevertebral ganglia, vagal and sympathetic visceral afferents that transmit sensory information from the gut wall to the CNS, and rectospinal afferent pathways. For clarity, many of the neuronal components of the ENS are left out of Fig. 4. The ENS is often referred to as the "little brain in the gut" because it contains all the necessary components (*e.g*., sensory cells, sensory neurons, interneurons and motor neurons) to independently initiate GI reflexes involved in peristalsis, secretion, absorption and transport of electrolytes or nutrients, local blood flow regulation and immune regulation. Functional abnormalities of the ENS and inputs from visceral efferent collateral of sympathetic spinal afferents are implicated in FGIDs and some of the GI symptoms treatable by the present disclosure.

Sympathetic spinal afferent pathways convey nociceptive information to the CNS from the viscera and the gastrointestinal tract. Therefore, the sympathetic spinal afferents run through the splanchnic nerves with their cell somas in the dorsal root ganglia synapsing with neurons in the dorsal horn of the spinal cord. From there, the signals are conveyed to higher centers in the brain. These sympathetic spinal afferents have axon collaterals that form en passant synapses with prevertebral ganglia (PVG) neurons, *i.e*., the inferior messenteric ganglia (IMG), superior mesenteric ganglia (SMG) and celiac ganglia (CG). Visceral spinal afferents are arranged in series with circular and longitudinal muscle layers, and respond to tension (*e.g*., form tension receptors in smooth muscles). Vagal afferents run through the nodose ganglia (NG) and jugular ganglia (JG) to the brainstem transmitting physiologic information. Reflex arcs and entero-enteric reflexes involving neuronal communication between the ENS and prevertebral ganglia (PVG) are described below.

Spinal and vagal afferents transmit sensory information from upper GI tract to brain and both vagal and spinal afferent fibers respond to mechanical stimulation (*e.g*., contraction and intraluminal distension). However, vagal afferents transit information within the physiological range. In contrast, some spinal afferents respond over a wide dynamic range extending into the noxious/pathophysiologic levels of distension. Therefore, these spinal endings transmit information about visceral pain. There are also other types of spinal afferents that respond only to pain (or noxious stimulation/or levels of distension or contraction). These include high-threshold mechanoreceptors that do not respond under normal physiological stimulation. These are referred to as silent nociceptors that can be activated by injury or mucosal inflammation of the GI tract.

Cell bodies of spinal (and some vagal) afferents are found in dorsal root ganglia (DRG) of the spinal nerves. Spinal afferents enter the spinal cord and make synaptic connections with second order neurons in the dorsal horns that send visceral/pain information to the brain. Afferent fibers travel in the spinothalamic and spinoreticular pathways. The former are thought to represent the major pathways for visceral pain. Spinal sensitization mechanisms following tissue injury results in hyperalgesia (a leftward shift in pain sensation), and an increase of the somatic referral area (receptive-field) referred to as allodynia, that can activate second order dorsal horn neurons of the spinal cord. A similar pattern is observed In FGIDs, where there is a leftward shift in the stimulation-pain curve and an increase in allodynia. As discussed below, brain-gut axis abnormalities involving CNS-ENS communication pathways can be selectively modulated to treat visceral pain and GI motility disorders associated with FGIDs. Neural mechanisms are an important component of FGIDs, and interventions such as spinal cord stimulation targeting modulation of these mechanisms at appropriate locations can be used to effectively treat severe abdominal visceral pain and GI motility disorders associated with FGIDs.

### Intestinofugal afferent neurons

IFANs relay mechanosensory information to the sympathetic prevertebral ganglion neurons. In contrast to visceral spinal afferents, IFANs detect changes in luminal volume, and are arranged in parallel to the circular muscle fibers and they respond to stretch of the muscle rather than tension. In PVG, IFANs release substance P and calcitonin gene related peptide (CGRP) that causes a slow excitatory postsynaptic potential (sEPSP) in sympathetic neurons. The release of these peptides is facilitated by release of neurotensin from central preganglionic nerves. Release of enkephalins from some central preganglionic nerves inhibits release of substance P (SP). Therefore, mechanosensory information that is transmitted to prevertebral ganglia via efferent axon collaterals of mechanosensory spinal afferents can be dually modulated in the prevertebral ganglia by neurotensin and enkephalins. IFANs are important because they form extended neural networks that connect the lower intestinal tract to the upper intestinal tract and coordinate entero-enteric reflexes over long distances in the GI tract. This is essential for normal transit and digestive functions of the bowels. IFANs also provide a protective buffer against large increase in tone and intraluminal pressure by eliciting a reflex-arc through the PVG to the gut wall to suppress circular muscle contraction and reduce smooth muscle tone.

### Intrinsic Primary Afferent Neurons

Intrinsic primary afferent neurons (IPANs) receive stimulatory signals (either mechanical or chemical in nature) from the gut lumen (anywhere in the GI tract), and activate interneurons or motor neurons of an extensive enteric neural network that coordinates all motor, secretory, absorptive and vasomotor reflexes through the enteric nervous system. In contrast, the extrinsic primary afferent neurons (EPANs) receive signals from the ENS, the smooth muscles and the gut mucosa, and transmit these signals to the CNS. In turn, the local activity of the enteric nervous system is modulated by efferent autonomic nervous system pathways (*e.g*., sympathetic efferent pathways depicted in Fig. 4) in response to EPANs.

### Efferent Collaterals of Sympathetic Spinal Afferents with SP/CGRP

It is noteworthy that CGRP is present in most splanchnic afferents, and that CGRP immunoreactivity is nearly absent from the gut after treatment with a sensory toxin capsaicin or after splanchnic nerve section, indicating its presence in visceral afferents. About 50% of CGRP- afferent neurons are shown to contain substance P and neurokinin-A. These mediators contribute to the development of visceral hyperalgesia in two important ways. First, CGRP/SP/NKA release at the spinal cord from central endings of primary afferents is important in the development of sensitization and visceral hyperalgesia. Therefore, release of neuropeptides from central collaterals contributes to painful sensations. Second, peripheral release of CGRP/SP/NKA can modify sensory inputs in FGIDs like IBS (or FD), thereby causing alterations in smooth muscle contractions, immune activation and mast cell degranulation, among others. Overall, efferent collaterals of sympathetic spinal afferents are involved in neural-immune activation of mast cells (other immune cells) and the enteric nervous system. This can create a vicious cycle that exacerbates pain sensation and GI motility/symptoms. In some instances of the present disclosure, sympathetic block by spinal cord stimulation may interfere with (*e.g*., minimize or prevent) immune activation and the vicious cycle of events.

### Other Sensitization Mechanisms of Visceral Hypersensitivity

Peripheral visceral nociceptive afferent pathways are involves in peripheral sensitization. Pro-inflammatory mediators can sensitize sympathetic spinal afferent fibers and contribute to visceral hypersensitivity and pain sensation. Mediators of sensitization include the sensory enterochromaffin cells (EC) in the gut mucosa and the immune mast cells (MC). EC cells sense mechanical or chemical stimuli from the lumen and, upon release of serotonin (5-HT) or ATP (among other mediators), activate intrinsic primary afferents to modulate gut reflexes, sympathetic spinal afferents to modulate sensation, or pain. Other peripheral sensitization mediators include 5-HT signaling pathways, purinergic pathways, voltage-gated sodium channels, protease activated receptor 2, transient receptor potential vallinoid receptors (VR1), other non-specific cation channels (NSCCs; P2X and 5HT3), bradykinin, adenosine, prostaglandins and lipooxygenase products.

Immune cell mediator release from mast cells (*e.g*., histamine, PG's, adenosine, tryptases, proteases, substance P, etc.) is also believed to contribute to sensitization and pain sensation in both IBS (no-inflammation present) and inflammatory bowel diseases (with inflammation present). EC and MC contain and release 5-HT involved in visceral sensation and modulation of GI motility. Drug interventions directed towards the 5-HT signaling pathway with 5HT₃ antagonists, 5HT₄ agonists and 5HT_{1A} antagonists, are of some benefit in the modulation of visceral pain and restoration of abnormal bowel function (habits) to more normal, but their success has been limited by adverse events and concerns over safety (*e.g*., tegaserod, a partial 5-HT₄ agonist for constipation predominant IBS patients has been discontinued due to potential life-threatening cardiac complications).

Other processes implicated in visceral pain and hypersensitivity in FGIDs may include abnormal ANS responses in descending modulation of visceral nociceptive pathways, stress responses and abnormal hypothalamic pituitary adrenal axis responses involving corticotropin releasing factor, aberrant central processing of visceral nociception (*e*.*g*., in the anterior cingulate cortex, brainstem and amygdala), and central visceral nociceptive afferent pathways. And, as described herein, neuromodulation devices can target the ANS (*e.g*., afferent or efferent sympathetic or parasympathetic limbs of the ANS) to reduce or alleviate GI symptoms depending on severity and progression of one or more FGIDs.

Examples of FGIDs treatable by the present disclosure are listed above and can also include: functional esophageal disorders (*e.g*., functional heartburn, functional chest pain of presumed esophageal origin, functional dysphagia and globus); functional gastroduodenal disorders, such as functional dyspepsia (*e.g*., postprandial distress syndrome and epigastric pain syndrome), belching disorders (*e.g*., aerophagia and unspecified excessive belching), nausea and vomiting disorders (*e.g*., chronic idiopathic vomiting, functional vomiting, and cyclic vomiting syndrome), and rumination syndrome; functional bowel disorders, such as unspecified functional bowel disorder; functional abdominal pain syndrome; functional gallbladder and Sphincter of Oddi (SO) disorders (*e.g*., functional gallbladder disorder, functional biliary SO disorder, and functional pancreatic SO disorder); functional anorectal disorders, such as functional fecal incontinence, functional anorectal pain (*e*.*g*., chronic proctalgia and proctalgia fugax), and functional defecation disorders (*e.g*., dyssynergic defecation and inadequate defecatory propulsion); childhood functional GI disorders in infants/toddlers, such as infant regurgitation, infant rumination syndrome, cyclic vomiting syndrome, infant colic, functional diarrhea, infant dyschezia and functional constipation; and childhood functional GI disorders in children/adolescents, such as vomiting and aerophagia (*e.g*., adolescent rumination syndrome, cyclic vomiting syndrome, and aerophagia), abdominal pain-related functional GI disorders (*e.g*., functional dyspepsia, IBS, abdominal migraine, and childhood functional abdominal pain syndrome), and constipation and incontinence (*e.g*., functional constipation and non-retentive fecal incontinence). Subjects treatable by the present disclosure can, in some instances, be diagnosed with (or suspected of having) a functional GI disorder as well as one or more related or unrelated medical conditions. Other examples of GI disorders/FGIDs treatable by the present disclosure are listed in Table 1.

**Table 1; GI Disorders, FGIDs, and associated symptoms treatable by the present disclosure**

| **GI Disorder/FGID/associated symptom(s)** |
|---|
| ▪ Irritable Bowel Syndrome (IBS) |
| • visceral pain associated with IBS |
| • GI discomfort associated with IBS |
| • abnormal bowel habits associated with IBS |
| • dysmotility associated IBS |
| • constipation predominant IBS (C-IBS) |
| • diarrhea predominant IBS (D-IBS) |
| • alternating constipation/diarrhea episodes (C/D-IBS) |
| • post-infectious IBS |
| ▪ Functional Dyspepsia (FD) |
| **•** gastric distress associated with FD |
| • abdominal pain associated with FD |
| • bloating associated with FD |
| • GI discomfort associated with FD |
| • gastroparesis associated with FD |
| ▪ Functional Abdominal Pain Syndrome |
| ▪ Belching Disorder (aerophagea) |
| ▪ Gastroparesis |
| • postprandial distress syndrome |
| • epigastric pain syndrome |
| ▪ Functional Esophageal Disorders |
| • non-cardiac chest pain (*e.g*., abnormal esophageal motility of esophageal spasm or nut-cracker esophagus) |
| • functional heart burn |
| • functional dysphagia and globus |
| ▪ IBS Secondary to Crohn's Disease |
| • CD-IBS, pain, and colonic hypersensitivity occurs during remission |
| ▪ Rumination Syndrome |
| ▪ Nausea and Vomiting Disorders |
| • chronic idiopathic vomiting syndrome |
| • functional vomiting syndrome |
| • cyclic vomiting syndrome |
| ▪ Functional Gallbladder and Sphincter of Oddi (SO) Disorders |
| • functional gallbladder disorder |
| • functional biliary SO disorder |
| ▪ Functional Anorectal Disorders |
| • functional fecal incontinence |
| ▪ Functional Anorectal Pain |
| • chronic proctalgia |
| • proctalgia fugax |
| ▪ Childhood Functional GI Disorders in Infants/Toddlers |
| • infant regurgitation |
| • infant rumination syndrome |
| • cyclic vomiting syndrome |
| • infant colic |
| • functional diarrhea |
| • infant dyschezia |
| • functional constipation |
| • pain and GI symptoms in autism |
| ▪ Childhood Functional GI Disorders in Children/Adolescents |
| • vomiting |
| • aerophagia |
| • adolescent rumination syndrome |
| • cyclic vomiting syndrome |
| ▪ Abdominal Pain Related Functional GI Disorders |
| • FD |
| • IBS |
| • abdominal migraine |
| • childhood functional abdominal pain syndrome |

In some instances, a therapy delivery device can be placed into electrical communication with an ANS and/or CNS nerve target that is associated with the functional GI disorder via an intravascular or intraluminal route. In other instances, a therapy delivery device can be placed into electrical communication with an ANS and/or CNS nerve target associated with the functional GI disorder target via a transcutaneous approach.

Examples of ANS nerve targets into which a therapy delivery device may be placed into electrical communication with can include, but are not limited to, any tissues of the SNS or the PNS. In some instances, ANS nerve targets into which a therapy delivery device may be placed into electrical communication with can include a sympathetic chain ganglion, an efferent of a sympathetic chain ganglion, or an afferent of a sympathetic chain ganglion. In other instances, the sympathetic chain ganglion can be a cervical sympathetic ganglion, a thoracic sympathetic ganglion, or a stellate ganglion. Examples of cervical sympathetic ganglia can include an upper cervical sympathetic ganglion, a middle cervical sympathetic ganglion, or a lower cervical sympathetic ganglion. Examples of thoracic sympathetic ganglia can include a T1 sympathetic ganglia, a T2 sympathetic ganglia, a T3 sympathetic ganglia, a T4 sympathetic ganglia, a T6 sympathetic ganglia, or a T7 sympathetic ganglia. Other examples of ANS nerve targets can include a mesenteric plexus or a gastric plexus.

Examples of CNS nerve targets into which a therapy delivery device may be placed into electrical communication with can include, but are not limited to, a C1, C2, C3, C4, C5, C6, C7, or C8 spinal cord segment or spinal nervous tissue associated therewith, a T1, T2, T3, T4, T5, T6, T7, T8, T9, T10, T11, or T12 spinal cord segment or spinal nervous tissue associated therewith, a L1, L2, L3, L4, or L5 spinal cord segment or spinal nervous tissue associated therewith, or a S1, S2, S3, S4, or S5 spinal cord segment or spinal nervous tissue associated therewith. In some instances, a CNS nerve target can include a ventral or dorsal root thereof.

After placing the therapy delivery device, the therapy delivery device can be activated to deliver a therapy signal (*e.g*., an electrical signal or magnetic field) to the ANS and/or CNS nerve target. In some instances, delivery of a therapy signal to the ANS and/or CNS nerve target can prevent a sign and/or symptom associated with the functional GI disorder from either increasing or decreasing (as compared to a control or baseline). In other instances, delivery of a therapy signal to the ANS and/or CNS nerve target can cause a sign and/or symptom associated with the functional GI disorder to decrease (as compared to a control or baseline). The therapy delivery device can be activated at the onset of an episode (*e.g*., the onset of a sign and/or symptom) associated with the functional GI disorder or, alternatively, the therapy delivery device can be activated continuously or intermittently to reduce or eliminate the frequency of such episode(s).

Delivery of the electrical signal to the ANS and/or CNS nerve target can affect central motor output, nerve conduction, neurotransmitter release, synaptic transmission, and/or receptor activation at the target tissue(s). For example, the ANS may be electrically modulated to alter, shift, or change sympathetic and/or parasympathetic activity from a first state to a second state, where the second state is characterized by a decrease in sympathetic and/or parasympathetic activity relative to the first state. As discussed above, delivery of an electrical signal to the ANS and/or CNS nerve target can, in some instances, substantially block activity of the autonomic nervous tissue target or spinal nervous tissue target. In other instances, delivery of an electrical signal to the ANS and/or CNS nerve target can achieve a complete nerve conduction block of autonomic nervous tissue target or spinal nervous tissue target for a desired period of time. In other instances, delivery of an electrical signal to the ANS and/or CNS nerve target can achieve a partial block of the autonomic nervous tissue target or spinal nervous tissue target for a period of time sufficient to decrease sympathetic and/or parasympathetic nerve activity. In further instances, delivery of an electrical signal to the ANS and/or CNS nerve target can increase sympathetic tone (*e.g*., from a hyposypmathetic state) to a normal or baseline level. The degree to which sympathetic and/or parasympathetic activity is decreased or increased can be titrated by one skilled in the art depending, for example, upon the nature and severity of the functional GI disorder.

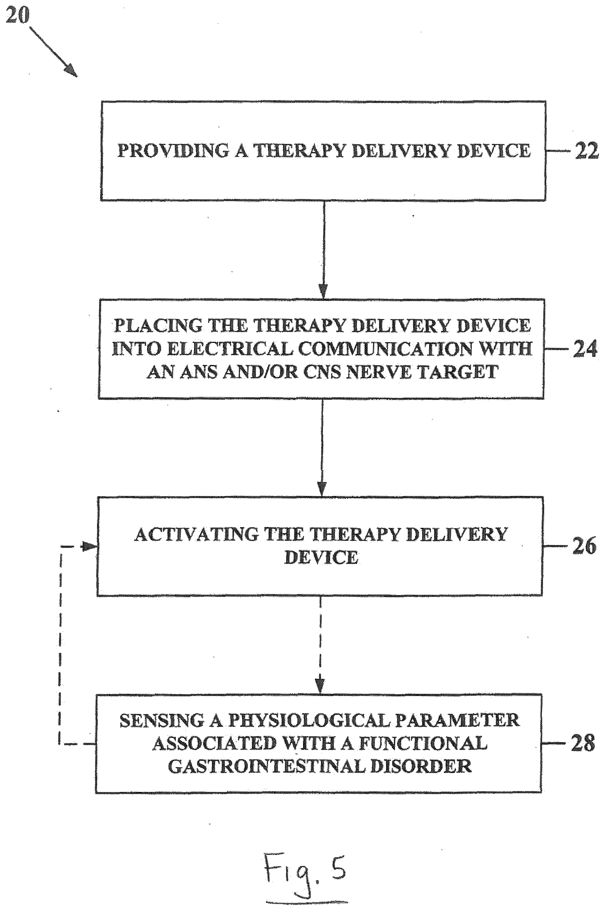

In another aspect, the present disclosure can include a method 20 (Fig. 5) for treating a functional GI disorder in a subject. One step of the method 20 can include providing a therapy delivery device (Step 22). Alternatively, Step 22 can include providing a closed-loop therapy delivery system. Examples of suitable therapy delivery devices (and systems) are described above and further illustrated below. At Step 24, the therapy delivery device (or system) can be placed into electrical communication (*e.g*., indirect electrical contact) with an ANS and/or CNS nerve target associated with the functional GI disorder. In some instances, "indirect electrical contact" can mean that the therapy delivery device (or system) is located adjacent or directly adjacent (but not in physical contact with) the ANS and/or CNS nerve target such that delivery of a therapy signal (*e.g*., an electrical signal or a magnetic field) can modulate a function, activity, and/or characteristic of the autonomic nervous tissue and/or spinal nervous tissue comprising the ANS and/or CNS nerve target.

In one example, Step 24 of the method 20 can include transvascular or transluminal delivery of an electrical energy to an ANS and/or CNS nerve target associated with the functional GI disorder. Thus, in some instances, the method 20 can include providing a therapy delivery device (or system) configured for transvascular or transluminal insertion and placement within the subject. For instance, a therapy delivery device configured for intravascular or intraluminal placement in a subject can include an expandable electrode as disclosed in the '331 application. The therapy delivery device can be inserted into a vessel or lumen of the subject. Non-limiting examples of vessel and lumens into which the therapy delivery device can be inserted include arteries, veins, an esophagus, a trachea, a vagina, a rectum, or any other bodily orifice. The therapy delivery device can be surgically inserted into the vessel or lumen via a percutaneous, transvascular, laparoscopic, or open surgical procedure.

After inserting the therapy delivery device into the vessel or lumen, the therapy delivery device can be advanced (if needed) to an intraluminal target site so that the therapy delivery device is in electrical communication with the ANS and/or CNS nerve target. In some instances, advancement of the therapy delivery device can be done under image guidance (*e.g*., fluoroscopy, CT, MRI, etc.). Intraluminal target sites can include intravascular or intraluminal locations at which the therapy delivery device can be positioned. For example, an intraluminal target site can include a portion of a vessel wall that is innervated by (or in electrical communication with) autonomic nervous tissue and/or spinal nervous tissue comprising the ANS and/or CNS nerve target (respectively). Examples of intraluminal target sites can include, without limitation, vascular or luminal sites innervated by and/or in electrical communication with any nervous tissue(s) of the SNS or PNS, such as neurons, axons, fibers, tracts, nerves, plexus, afferent plexus fibers, efferent plexus fibers, ganglion, pre-ganglionic fibers, post-ganglionic fibers, a mesenteric plexus, a gastric plexus, cervical sympathetic ganglia/ganglion, thoracic sympathetic ganglia/ganglion, afferents thereof, efferents thereof, a sympathetic chain ganglion, a thoracic sympathetic chain ganglion, an upper cervical chain ganglion, a lower cervical ganglion, an inferior cervical ganglion, and a stellate ganglion.

After placing the therapy delivery device, a therapy signal (*e.g*., an electrical signal or a magnetic field) can be delivered to the ANS and/or CNS nerve target. The therapy signal can be delivered in an amount and for a time sufficient to effectively treat the functional GI disorder.

In one example, the method 20 can be employed to treat a functional GI disorder, such as functional dyspepsia or functional constipation. In such instances, a therapy delivery device can be inserted into a vessel of the subject and then advanced to a point substantially adjacent an intraluminal target site of the ANS, such as a mesenteric plexus, a gastric plexus, or a ganglion of the SNS. Alternatively, a therapy delivery device can be inserted into a vessel of the subject and then advanced to a point substantially adjacent an intraluminal target site of the CNS, such as a spinal cord segment, a dorsal root thereof, or a ventral root thereof. Next, the therapy delivery device can be activated to deliver a therapy signal to the intraluminal target site in an amount and for a time sufficient to effect a change in sympathetic and/or parasympathetic activity in the subject and thereby treat the functional dyspepsia or functional constipation.

In another aspect, the method 20 can include providing a therapy delivery device (or system) configured for placement on the skin of the subject. In some instances, a device for transcutaneous delivery of a therapy signal can comprise a housing configured for placement on (*e.g*., directly on) the skin of a subject so that the therapy delivery device is in electrical communication with an ANS and/or CNS nerve target associated with a functional GI disorder (*e.g*., the device is especially configured to directly overlie the nerve target), at least one electrode connected to the housing and configured to deliver an electrical signal to the nerve target, and a power source in electrical communication with the at least one electrode. Examples of therapy delivery devices configured for transcutaneous delivery of one or more therapy signals are disclosed above and described in more detail below. In some instances, a therapy delivery device (or system) can be positioned about the subject, without penetrating the skin of the subject, so that the therapy delivery device is in electrical communication with an ANS and/or CNS nerve target associated with a functional GI disorder. Non-limiting examples of ANS and CNS nerve targets into which the therapy delivery device can be placed into electrical communication are described above. After placing the therapy delivery device (or system), a therapy signal can be delivered to the ANS and/or CNS nerve target. The therapy signal can be delivered in an amount and for a time sufficient to effectively treat the functional GI disorder.

In one example, the method 20 can include treating a functional GI disorder, such as functional dyspepsia, functional constipation, or GERD. A therapy delivery device can be placed, without penetrating the skin of the subject, into electrical communication with an ANS nerve target associated with functional dyspepsia, functional constipation or GERD, such as a mesenteric plexus, a gastric plexus, or a ganglion of the SNS. Next, the therapy delivery device can be activated to deliver a therapy signal to the ANS nerve target in an amount and for a time sufficient to effect a change in sympathetic and/or parasympathetic activity in the subject and thereby treat the functional dyspepsia, functional constipation or GERD.

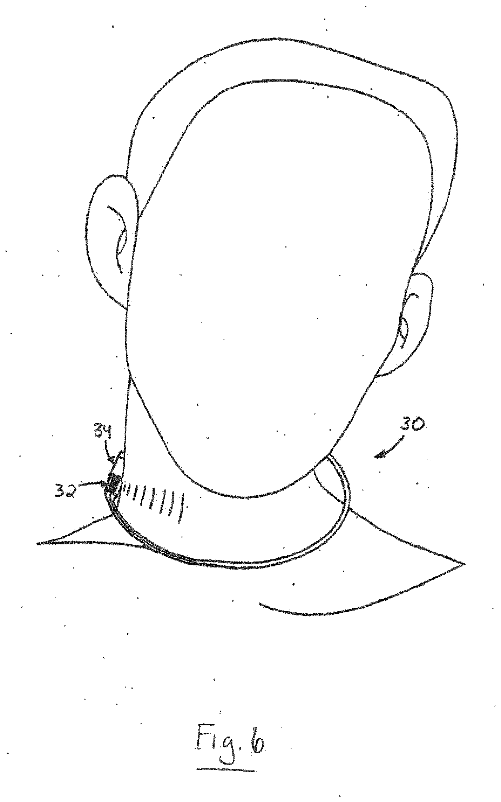

In another example, a transcutaneous neuromodulation device can comprise a wearable accessory item, such as a necklace or collar 30 (Fig. 6). As shown in Fig. 6, a necklace or collar 30 can be configured to include at least one electrode 32 for delivering a therapy signal to a particular region of a subject's neck (*e.g*., an anterior or posterior region thereof) depending upon the desired neuromodulatory effect. The necklace or collar 30 can additionally include an integral power source 34 (*e.g*., a rechargeable battery). It will be appreciated that the electrode(s) 32 can alternatively be powered by a wireless power source (not shown). The necklace or collar 30 can be configured to obtain a pre-selected position about a subject's neck by, for example, using a positioning guide (not shown), weighting the necklace or collar, etc. Alternatively, the subject can manually adjust the necklace or collar 30 as needed to optimize delivery of the therapy signal from the electrode(s) 32 to an ANS and/or CNS nerve target.

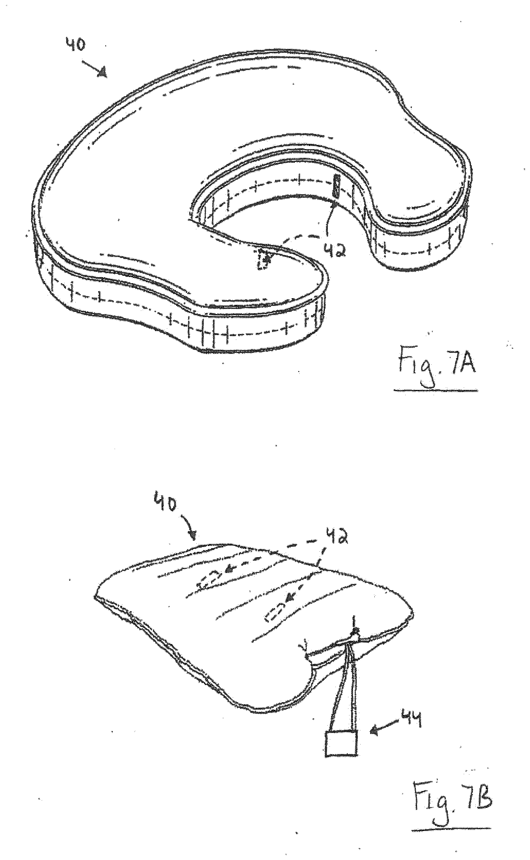

In another example, a transcutaneous neuromodulation device can comprise a pillow 40 (Figs. 7A-B). In some instances, the pillow 40 (Fig. 7A) can be configured as a collar for use in a reclined or upright position, such as on an airplane, in a car, on a couch, etc. The pillow 40 can include at least one electrode 42 configured to deliver a therapy signal to an ANS and/or CNS nerve target (*e.g*., in a subject's head or neck). As shown in Fig. 7A, the pillow 40 includes two oppositely disposed electrodes 42. The pillow 40 can also include a power source (not shown), which may be integrally connected with the pillow or located remotely (*i.e*., wirelessly) therefrom. In other instances, the pillow 40 (Fig. 7B) can comprise a traditional or conventional pillow for use when a subject is sleeping or lying in bed. As shown in Fig. 7B, the pillow 40 can include two oppositely disposed electrodes 42 configured to deliver a therapy signal to a target nerve when the subject neck or head is straddled between the electrodes. The pillow 40 can further include a power source 44 that is in direct electrical communication with the electrodes 42; however, it will be appreciated that the power source can be located remotely (*i.e*., wirelessly) from the pillow.

It will be appreciated that the transcutaneous neuromodulation devices illustrated in Figs. 6 and 7A-B are illustrative only and, moreover, that such devices can include any wearable item, accessory, article of clothing, or any object, device, or apparatus that a subject can use and, during use, comes into close or direct contact with a portion of the subject's body (*e.g*., the subject's neck). Examples of such transcutaneous neuromodulation devices can include vests, sleeves, shirts, socks, shoes, underwear, belts, scarves, wrist bands, gloves, ear pieces, band-aids, turtle neck, pendants, buttons, earrings, stickers, patches, bio-films, skin tattoos (*e.g*., using neuro-paint), chairs, computers, beds, head rests (*e.g*., of a chair or car seat), cell phones, and the like.

From the above description of the present disclosure, those skilled in the art will perceive improvements, changes and modifications. Such improvements, changes, and modifications are within the skill of those in the art and are intended to be covered by the appended claims. All patents, patent applications, and publication cited herein are incorporated by reference in their entirety.

### Alternative Aspects (these are not claims)

1. A therapy delivery device for treating a functional gastrointestinal (GI) disorder in a subject the device comprising:
   a housing configured for placement on the skin of the subject;
   at least one electrode connected to the housing and configured to deliver an electrical signal to an autonomic nervous system (ANS) target associated with the functional GI disorder, the ANS nerve target including one or more of a mesenteric plexus, a gastric plexus, or a ganglion of the sympathetic nervous system (SNS); and
   a power source in electrical communication with the at least one electrode;
   wherein the functional GI disorder is at least one of the functional dyspepsia, functional constipation, and gastroesophageal reflux disease.
2. The device of alternative aspect 1, being configured as a wearable accessory item.
3. The device of alternative aspect 2, wherein the wearable accessory item is a collar or necklace.
4. The device of alternative aspect 1, being configured as a pillow.
5. The device of alternative aspect 1, further including:
   a sensing component configured to detect at least one physiological parameter associated with the functional GI disorder, and
   a controller configured to automatically coordinate operation of the power source and the sensing component;
   wherein the controller is configured to direct delivery of the electrical signal to the at least one electrode to modulate activity at the ANS nerve target.
6. The device of alternative aspect 5, wherein the at least one physiological parameter is a chemical moiety or an electrical activity.
7. A closed-loop therapy delivery system for treating a functional GI disorder in a subject, the system comprising;
   a sensing component configured to detect at least one physiological parameter associated with the functional GI disorder;
   a delivery component configured for placement on the skin of the subject adjacent an ANS target associated with the functional GI disorder, the ANS nerve target including one or more of a mesenteric plexus, a gastric plexus, or a ganglion of the SNS; and
   a controller configured to automatically coordinate operation of the sensing and delivery components;
   wherein the controller is configured to deliver an electrical signal to the delivery component in an amount and for a time sufficient to treat at least one of functional dyspepsia, functional constipation, and gastroesophageal reflux disease.
8. The system of alternative aspect 7, wherein the controller is in electrical communication with the power source.
9. The system of alternative aspect 7, wherein the at least one physiological parameter includes a chemical moiety or an electrical moiety.
10. The system of alternative aspect 7, wherein the delivery component comprises a housing and at least one electrode connected to the housing, the housing being configured as a wearable accessory item.
11. The system of alternative aspect 10, wherein the wearable accessory item is a collar or necklace.
12. The system of alternative aspect 10, wherein the wearable accessory item is a pillow.
13. The system of alternative aspect 12, wherein the at least one physiological parameter is a chemical moiety or an electrical activity.
14. A closed-loop therapy delivery system for treating a functional GI disorder in a subject, the system comprising:
   a sensing component configured to detect at least one physiological parameter associated with the functional GI disorder;
   a delivery component configured for placement in a vessel of the subject at a point substantially adjacent an intraluminal target site of the ANS, the central nervous system (CNS), or both, that is associated with the functional GI disorder; and
   a controller configured to automatically coordinate operation of the sensing and delivery components;
   wherein the controller is configured to deliver an electrical signal to the delivery component in an amount and fur a time sufficient to treat at least one of functional dyspepsia and functional constipation.
15. The system of alternative aspect 14, wherein the therapy signal is electrical energy.
16. The system of alternative aspect 14, wherein the at least one physiological parameter is a chemical moiety or an electrical activity.

## Claims

1. A closed-loop therapy delivery system for treating a functional GI disorder in a subject, the system comprising:
a sensing component configured to detect at least one physiological parameter associated with the functional GI disorder;
a delivery component configured for placement on the skin of t e subject adjacent an ANS target associated with the functional GI disorder, the ANS nerve target including one or more of a mesenteric plexus, a gastric plexus, or a ganglion of the SNS; and
a controller configured to automatically coordinate operation of the sensing and delivery components;
wherein the controller is configured to deliver an electrical signal to the delivery component in an amount and for a time sufficient to treat at least one of functional dyspepsia, functional constipation, and gastroesophageal reflux disease.

2. The system of claim 1, wherein the controller is in electrical communication with the power source.

3. The system of claim 1, wherein the at least one physiological parameter includes a chemical moiety or an electrical moiety.

4. The system of claim 1, wherein the delivery component comprises a housing and at least one electrode connected to the housing, the housing being configured as a wearable accessory item.

5. The system of claim 4, wherein the wearable accessory item is a collar or necklace.

6. The system of claim 4, wherein the wearable accessory item is a pillow.

7. The method of claim 6, wherein the at least one physiological parameter is a chemical moiety or an electrical activity.

8. A closed-loop therapy delivery system for treating a functional GI disorder in a subject, the system comprising:
a sensing component configured to detect at least one physiological parameter associated with the functional GI disorder;
a delivery component configured for placement in a vessel of the subject at a point substantially adjacent an intraluminal target site of the ANS, the central nervous system (CNS), or both, that is associated with, the functional GI disorder; and
a controller configured to automatically coordinate operation of the sensing and delivery components;
wherein the controller is configured to deliver an electrical signal to the deliver component in an amount and for a time sufficient to treat at least one of functional dyspepsia and functional constipation.

9. The system of claim 8, wherein the therapy signal is electrical energy.

10. The system of claim 8, wherein the at least one physiological parameter is a chemical moiety or an electrical activity.
